# EUROPÄISCHE PATENTSCHRIFT

(11) **EP 1 671 661 B1**
(45) Veröffentlichungstag und Bekanntmachung des Hinweises auf die Patenterteilung: **15.07.2009**
(21) Anmeldenummer: 05025607.2
(22) Anmeldetag: 24.11.2005
(51) Int. Cl.: A61L 27/02, A61L 27/54

(54) **Antibiotikum/Antibiotika enthaltendes Knochenersatzmaterial mit retardierender Wirkstofffreisetzung**
Antibiotic-containing bone replacement material with retarding drug release
Matériau pour le comblement osseux ayant une libération retardée d'antibiotiques

(30) Priorität: 15.12.2004 DE 102004060666
(43) Veröffentlichungstag der Anmeldung: 21.06.2006
(73) Patentinhaber: Heraeus Kulzer GmbH, 63450 Hanau (DE)
(72) Erfinder: Kühn, Klaus-Dieter, Dr., 35041 Marburg (DE); Vogt, Sebastian, Dr., 99084 Erfurt (DE)
(74) Vertreter: Kühn, Hans-Christian

(56) Entgegenhaltungen:
- EP-A- 0 985 413
- EP-A1- 1 374 854
- EP-A2- 1 374 923
- WO-A-01/76649
- US-A- 5 681 873

## Beschreibung

Die Osteomyelitis ist eine sehr schwer zu therapierende Infektion des Knochengewebes. Üblicherweise erfolgt die Behandlung durch ein Debridement der infizierten Knochenbereiche. Bewährt hat sich eine anschließende lokale Antibiotika-Therapie mit nicht resorbierbaren Septopal^{®}-Ketten oder mit Gentamicin enthaltenden Kollagenvliesen. Dabei wird Gentamicin lokal in hohen Dosen feigesetzt, das die verblieben bakteriellen Keime abtötet. Wünschenswert ist ein Knochenersatzmaterial, dass einerseits Antibiotika lokal freisetzen kann und das andererseits das Knochenwachstum begünstigt und eine Platzhalterfunktion hat.

Die Verwendung von Calciumsulfat-Dihydrat als Knochenersatzmaterial ist seit langem bekannt (E. Martin: Zur Ausfüllung von Knochenhöhlen mit totem Material. Zentalb Chir 21 (1894) 193-200; E. Edberg: Some experiences of filling osteous cavities with plaster. Acta Chir Scand 67 (1930) 313-319; J. O. Hollinger, J. Brekke, E. Kruskin, D. Lee: Role of bone substiutes. Clin Orthop 324 (1996) 55-65).

In den Patenten US2002110541, US5807567, US2002197315, US6652887, US5756127 und US5614206 wurden Knochenersatzmaterialien beschrieben, die im wesentlichen aus einer Mischung aus α- und β-Calciumsulfat bestehen und als Drug- Delivery-Systeme für pharmazeutische Wirkstoffe verwendet werden sollen. Diese Knochenersatzmaterialien setzten die Hauptmenge des inkorporierten Wirkstoffes in den ersten Stunden nach Einbringung des Materials in eine wässrige Lösung frei und danach werden nur noch geringe Wirkstoffmengen über einen Zeitraum von mehreren Tagen abgegeben.

In DE19953771 wurde ein Knochenersatzmaterial offenbart, das aus einem Gemisch aus Calciumsulfat-Dihydrat und nanopartikulärem Hydroxylapatit besteht. Formkörper aus diesem Material besitzen eine hohe innere Oberfläche und können mit wässrigen Antibiotikum-Lösungen getränkt werden. Die so behandelten Formkörper setzen das Antibiotikum mehr oder weniger verzögert frei. Die Hauptmenge des Antibiotikums wird in den ersten Stunden feigesetzt. Die Retardierung beruht auf der Adsorption des Wirkstoffes an der inneren Oberfläche der Formkörper.

In DE10114244, DE10114245, DE10114364, DE10227914 oder DE10227938 werden Wirkstoffformulierungen bzw. Verfahren zur Herstellung dieser Wirkstoffformulierungen beschrieben, die auf der Bildung bzw. der Verwendung von in Wasser gering löslichen Wirkstoffsalzen beruht. So wird in DE10227914 eine pharmazeutische Zubereitung mit retardierender Wirkstoffreisetzung offenbart, die aus Gemischen von pulverförmigen Teicoplanin und mindestens einer pulverförmigen, wasserlöslichen Salzform des Gentamicins, des Clindamycins, des Vancomycins, des Moxifloxacins,und des Ciprofloxacins und eines anorganischen Hilfsstoffes und/oder organischen Hilfsstoffes besteht. Als Hilfsstoffe werden unter anderem Calciumcarbonat, Calciumsulfat-Dihydrat, Tricalciumphosphat und Hydroxylapatit vorgeschlagen. Die pharmazeutische Zubereitung wird in Form von Tabletten, Formkörpern, Fäden und Granulaten als Implantatmaterial verwendet.

In DE10227935 wird eine antibiotische Beschichtung von porösen Körpern und deren Verwendung beschrieben. In das Porensystem von nichtmetallischen und metallischen porösen Körpern wird eine Beschichtung aus mindestens einem in Wasser gering löslichen Antibiotika-Salz aus der Gruppe der Fettsäuresalze und Dodecylsulfate des Netilmicins, des Sisomicins, des Gentamicins, des Clindamycins, des Amikacins, des Kanamycins, des Tobramycins, des Ciprofloxacins und des Vasncomycins eingebracht. Die Beschichtung wird auch auf poröse Pulver, poröse Granulate, auf poröse Formkörper und auf poröse Schichten von kompakten Körpern aufgebracht. Die antibiotisch beschichteten Körper sollen als Implantate verwendet werden.

Im US5055307 werden Granulate zur verzögerten Wirkstofffreisetzung vorgeschlagen. Diese Granulate sind aus einem Calciumphosphat mit einem Stoffmengenverhältnis von Ca zu P von 1,3 bis 1,8 gebildet, das eine Porosität von 0,1 % bis 70 % hat und eine spezifische Oberfläche von 0,1 m²/g bis 50 m²/g besitzt. Der Porengröße liegt im Bereich von 1 nm bis 10 µm. Die Granulate werden in einem Calcinierungsprozess im Temperaturbereich von 200 °C bis 1400 °c gebildet. Anschließend werden die Granulate mit einem Wirkstoff imprägniert. Es ist auch möglich, die Granulate mit einer Polymerlösung zu tränken und eine Polymerschicht auf den Granulaten abzuscheiden.

Dokument D1 (WO 01/76649) beschreibt ein Knochenersatzmaterial von Calciumsulfat und Calciumphospat, mit pharmazeutischem Wirkstoff wie ein Antibiotikum, und übergezogen mit einer Schicht von biokompatiblen Ölen oder Fette wie Glycerintripalmitat oder Glycerintristearat (vgl. D1, Seite 1, Zeilen 4, 5; Seite 7, Zeilen 15-22; Seite 8, Zeilen 17-24).

Dokument D2 (US5681873) offenbart eine biokompatible, pharmazeutische Komposition zur Behandlung eines Knochen- oder Gewebedefektes. Die Komposition enthält ein thermoplastisches Polymer, einen Kristallisationsstoff wie Calciiumsulfat oder Calciumcarbonat, einen biologischen aktiven Wirkstoff, zum Beispiel ein Antibiotikum, und einen Stoff zur verzögerten Freisetzung des Antibiotikums wie Glycerinstearat oder Glycerinpalmitat (vgl. D2, Spalte 1, Zeile 53- Spalte 2, Zeile 57; Spalte 7, Zeile 4 - Spalte 9, Zeile 37; Ansprüche 1, 5-7, 9, 10).

Die in WO0176649 und US5681873 beschriebenen Materialien sind allerdings nicht so verdichtet oder verpresst, dass die Granula zu einem kompakten Körper, der keine Makroporen mit Porendurchmesser größer 50 µm besitzt, verpresst sind.

Der Erfindung liegt die Aufgabe zu Grunde, ein kostengünstiges, einfach herstellbares Antibiotikum/Antibiotika enthaltendes Knochenersatzmaterial zu entwickeln, dass eine retardierte Wirkstofffreisetzung zeigt.

Die Aufgabe wurde erfindungsgemäß durch die Merkmale des Anspruchs 1 gelöst. Vorteilhafte Ausgestaltungen sind in den Unteransprüchen angegeben. Es wurde ein Antibiotikum/Antibiotika enthaltendes Knochenersatzmaterial gefunden, dadurch charakterisiert ist, dass es aus einem verdichteten Gemisch von Granula des Calciumsulfat-Dihydrats, des Calciumcarbonats und mindestens eines Vertreters der Aminoglykosid-Antibiotika, der Lincosamid-Antibiotika, der Glykopeptid-Aantibiotika, der Makrolid-Antibiotika, der Ketolid-Antibiotika, der Nitroimidazole, der Fluorchinolon-Antibiotika und der Oxazolidinon-Antibiotika, der Steroid-Antibiotika und der fungiziden/fungistatischen Antibiotika aufgebaut ist, die mit einer Schicht aus Glycerintripalmitat und/oder Glycerintristearat und/oder Glycerintrilaurat und/oder 1-Hexadecylalkohol teilweise oder vollständig umhüllt sind, welche die Granula miteinander verbindet. Das bedeutet, die einzenen Granula sind mit einer Schicht aus Glycerintripalmitat und/oder Glycerintristearat und/oder Glycerintrilaurat und/oder 1-Hexadecylalkohol teilweise oder vollständig umhüllt. Die Schicht hat eine bevorzugte Schichtdicke kleiner 100 µm.

Überraschend setzen die erfindungsgemäßen Formkörper Antibiotika über einen Zeitraum von mehreren Tagen bis zu zwei Wochen verzögert frei. Unter dem Begriff verdichtetes Gemisch wird verstanden, dass die Granula zu einem kompakten Körper, der keine Makroporen mit Porendurchmesser größer 50 µm besitzt, verpresst sind. Dazu sollte mit hoher Presskraft von mindestens 30kN (zum Beispiel bei einem Formkörper mit einem Durchmesser von etwa 6 mm) gepreßt werden. Die verdichtete Struktur beeinflußt die Diffusion der Wirkstoffe aus dem Inneren des Materials an die Oberfläche.

Anstelle von Glycerintrilaurat, Glycerintripalmitat und Glycerintristearat kann auch Glycerindilaurat, Glycerintrimyristat, Glycerindimyristat, Glycerindipalmitat, Glycerindistearat, Bienenwachs, Talg, Hartfett oder hydrierte Pflanzenöle eingesetzt werden.

Es werden die Wirkstoffe Gentamicin, Tobramycin, Netilmicin, Sisomycin, Amikacin, Lincosamin, Clindamycin, Teicoplanin, Vancomycin, Ramoplanin, Erythromycin, Telithromycin, Azithromycin, Ciprofloxcin, Moxifloxacin, Linezolid, Metronidazol, Tinidazol, Fluconazol, Amphotericin B, Nystatin, Griseofulvin, Fusidinsäure, Fosfomycin, Rifampicin, Chloramphenicol, Chlorhexidin, Polyhexanid oder deren als Einzelsubstanzen oder deren Gemische als Bestandteil der erfindungsgemäßen Knochenersatzmaterialien bevorzugt. Die Wirkstoffe können als in Wasser leicht lösliche Salze oder auch als in Wasser gering lösliche Salze eingesetzt werden. Deshalb ist es auch im Sinne der Erfindung, dass in Wasser gering lösliche Salze dieser Antibiotika in den erfindungsgemäßen Knochenersatzmaterialien inkorporiert sind. So zum Beispiel Gentamicinpalmitat, Tobramycinpalmitat und Gentamicindodecylsulfat.

Weiterhin ist es vorteilhaft, dass das Knochenersatzmaterial die Form von zylindrischen oder facettierten Tabletten oder die Form von Granulat mit einem Partikeldurchmesser von 100-2500 µm hat, die an der Oberfläche eine geschlossene Schicht aus Glycerintripalmitat und/oder Glycerintristearat und/oder Glycerintrilaurat und/oder 1-Hexadecylalkohol besitzt.

Es ist zweckmäßig, dass das Calciumcarbonat teilweise oder vollständig durch β-Tricalciumphosphat, Calciumhydrogenphosphat, Calciumhydrogenphosphat-Dihydrat, Magnesiumcarbonat, Trimagnesiumphosphat, Magnesiumhydroxid, Magnesiumoxid ersetzt wird.

Außerdem ist zweckmäßig, dass eine Zusammensetzung mit
50,0-90,0 Masseprozent Calciumsulfat-Dihydrat,
1,0-40,0 Masseprozent Calciumcarbonat,
1,0-40,0 Masseprozent Glycerintripalmitat und/oder Glycerintristearat und/oder Glycerintrilaurat und/oder 1-Hexadecylalkohol sowie
0,1-10,0 Masseprozent mindestens eines Vertreters der Aminoglykosid-Antibiotika, der Lincosamid-Antibiotika, der Makrolid-Antibiotika, der Ketolid-Antibiotika, der Nitroimidazole, der Fluorchinolon-Antibiotika, der Oxazolidinon-Antibiotika, der Steroid-Antibiotika, der Antiseptika und der fungiziden/fungistatischen Antibiotika bevorzugt wird.
Erfindungsgemäß sind Formkörper aus dem Knochenersatzmaterial gebildet, die vorzugsweise die Form von Kugeln, sphärischen Körpern, Ringen oder Zylindern, gegebenenfalls mit einer Durchbohrung, aufweisen.

Die Erfindung wird durch die nachstehenden Beispiele erläutert, ohne sie jedoch dadurch einzuschränken.

### Beispiel 1:

Es werden 179,91 g Calciumsulfat-Dihydrat (Merck), 44,98 g Calciumcarbonat (Fluka) und 6,88 g Gentamicinsulfat (AK545, entspricht 3,75 g Gentamicinbase) mit einer Rollmühle miteinander vermahlen. Anschließend wird dieses Gemisch mit einer auf 60 °C temperierten Lösung von 18,23 g Tripalmitin (Glycerintripalmitat von Fluka) in Ethanol vermischt. Es entsteht ein Gemisch von breiartiger Konsistenz. Nach Verdunsten des Ethanols wird das ausgehärtete Gemisch zu Granulat gebrochen und gesiebt. Dieses Granulat (Kornfraktion 63-500 µm) wird in einer üblichen Exzenter-Tablettiermaschine zu Tabletten mit einer Masse von 250 mg, einem Durchmesser von 6 mm und einer Höhe von 4,5 mm gepresst.

### Beispiel 2:

Es werden 176,51 g Calciumsulfat-Dihydrat (Merck), 44,13 g Calciumcarbonat (Fluka) und 11,47 g Gentamicinsulfat (AK 545, entspricht 6,25 g Gentamicinbase) mit einer Rollmühle miteinander vermahlen. Anschließend wird dieses Gemisch mit einer auf 60 °C temperierten Lösung von 17,89 g Tripalmitin (Glycerintripalmitat von Fluka) in Ethanol vermischt. Es entsteht ein Gemisch von breiartiger Konsistenz. Nach Verdunsten des Ethanols wird das ausgehärtete Gemisch zu Granulat gebrochen und gesiebt. Dieses Granulat (Kornfraktion 63-500 µm) wird in einer üblichen Exzenter-Tablettiermaschine zu Tabletten mit einer Masse von 250 mg, einem Durchmesser von 6 mm und einer Höhe von 4,5 mm gepresst.

### Beispiel 3:

Es werden 172,11 g Calciumsulfat-Dihydrat (Merck), 43,03 g Calciumcarbonat (Fluka), 7,81 g Gentamicinsulfat (Fujian, AK 640, entspricht 5,0 g Gentamicnbase) und 5,77 g Clindamycinhydrochlorid (Upjohn, AK867, entspricht 5,0 g Clindamycinbase) mit einer Rollmühle miteinander vermahlen. Anschließend wird dieses Gemisch mit einer auf 60 °C temperierten Lösung von 21,28 g Tripalmitin (Glycerintripalmitat von Fluka) in Ethanol vermischt. Es entsteht ein Gemisch von breiartiger Konsistenz. Nach Verdunsten des Ethanols wird das ausgehärtete Gemisch zu Granulat gebrochen und gesiebt. Dieses Granulat (Kornfraktion 63-500 µm) wird in einer üblichen Exzenter-Tablettiermaschine zu Tabletten mit einer Masse von 250 mg, einem Durchmesser von 6 mm und einer Höhe von 4,5 mm gepresst.

### Freisetzungsuntersuchungen

Es wurden jeweils 6 Tabletten der Beispiele 1 und 2 in 20 ml destilliertem Wasser bei 37 °C eingelagert. Täglich wurden 15 ml Feisetzungsmedium entnommen und durch frisches destilliertes Wasser ersetzt. Im entnommenen Medium wurde das freigesetzte Gentamicin bestimmt. Die Bestimmung des freigesetzten Gentamicins erfolgte mit einem TDX-Analyser der Firma Abbott. Bei der Berechnung der freigesetzten Gentamicin-Menge wurde die nicht vollständige Entnahme des Freisetzungsmediums ab dem ersten Tag mit berücksichtigt. Die jeweils freigesetzte Masse an Gentamicinbase von 6 Tabletten wurde in der nachfolgenden Tabelle in Abhängigkeit von der Lagerungszeit der Probkörper im Freisetzungsmedium dargestellt. Nach dem 11 Tag wurden die Freisetzungsversuche abgebrochen.

| | Gentamicinbase-Freisetzung von 6 Tabletten [µg] | | | | | | | | | |
|---|---|---|---|---|---|---|---|---|---|---|
| Zeit | 1 d | 2 d | 3 d | 4 d | 7 d | 8 d | 9 d | 10d | 11 d | 11 d |
| Tabletten | | | | | | | | | | |
| Beispiel 1 | 7.514 | 6.638 | 4.440 | 2.019 | 2.074 | 326 | 244 | 219 | 219 | 204 |
| Tabletten | | | | | | | | | | |
| Beispiel 2 | 13.877 | 12.388 | 6.781 | 3.668 | 2.617 | 369 | 334 | 206 | 207 | 256 |

## Patentansprüche

1. Antibiotikum/Antibiotika enthaltendes Knochenersatzmaterial mit retardierender Wirkstofffreisetzung, **dadurch gekennzeichnet, dass** es aus einem verdichteten Gemisch von Granula auf Basis von Calciumsulfat-Dihydrat und mindestens einem Mitglied der Gruppe bestehend aus Calciumcarbonat, β-Tricalciumphosphat, Calciumhydrogenphosphat, Calciumhydrogenphosphat-Dihydrat, Magnesiumcarbonat, Trimagnesium-phosphat, Magnesiumhydroxid und Magnesiumoxid
und mindestens eines Vertreters der Aminoglykosid-Antibiotika, der Lincosamid-Antibiotika, der Glykopeptid-Antibiotika, der Makrolid-Antibiotika, der Ketolid-Antibiotika, der Nitroimidazole, der Fluorchinolon-Antibiotika, Oxazolidinon-Antibiotika, der Steroid-Antibiotika, der Antiseptika und der fungiziden/fungistatischen Antibiotika gebildet ist,
wobei die Granula mit einer Schicht aus Glycerintripalmitat und/oder Glycerintristearat und/oder Glycerintrilaurat und/oder 1-Hexadecylalkohol teilweise oder vollständig umhüllt sind, welche die einzelnen Granula miteinander verbindet, und die Granula zu einem kompakten Körper, der keine Makroporen mit Porendurchmesser größer 50 µm besitzt, verpresst sind.

2. Antibiotikum/Antibiotika enthaltendes Knochenersatzmaterial nach Anspruch 1, **dadurch gekennzeichnet, dass** es die Form von Tabletten aus Granula mit einem Partikeldurchmesser von 100 bis 2500 µm oder die Form von Granulat aus Granula mit einem Partikeldurchmesser von 100 bis 2500 µm hat.

3. Knochenersatzmaterial nach Anspruch 2, wobei die Tabletten zylindrisch oder facettiert sind.

4. Antibiotikum/Antibiotika enthaltendes Knochenersatzmaterial nach einem der Ansprüche 1 bis 3 **gekennzeichnet durch** eine Zusammensetzung mit
50,0-90,0 Masseprozent Calciumsulfat-Dihydrat,
1,0-40,0 Masseprozent Calciumcarbonat,
1,0-40,0 Masseprozent Glycerintripalmitat und/oder Glycerintristearat und/oder Glycerintrilaurat und/oder 1-Hexadecylalkohol und
0,1-10,0 Masseprozent mindestens eines Vertreters derAminoglykosid-Antibiotika, der Lincosamid-Antibiotika, der Glykopeptid-Antibiotika, der Makrolid-Antibiotika, der Ketolid-Antibiotika, der Nitroimidazole, der Fluorchinolon-Antibiotika und der Oxazolidinon-Antibiotika, der Antiseptika, der Steroid-Antibiotika und derfungistatischen/fungiziden Antibiotika.

5. Antibiotikum/Antibiotika enthaltendes Knochenersatzmaterial nach einem der Ansprüche 1 bis 4, **dadurch gekennzeichnet, dass** der Vertreter Gentamicin, Tobramycin, Netilmicin, Sisomycin, Amikacin, Lincosamin, Clindamycin, Teicoplanin, Vancomycin, Ramoplanin, Erythromycin, Telithromycin, Azithromycin, Ciprofloxcin, Moxifloxacin, Linezolid, Metronidazol, Tinidazol, Fluconazol, Amphotericin B, Nystatin, Griseofulvin, Fusidinsäure, Fosfomycin, Rifampicin, Chloramphenicol, Chlorhexidin, Polyhexanid ist.

## Claims

1. Antibiotic/antibiotics containing bone substitute material with sustained active substance release **characterized in that** it is composed of a compacted mixture of granules on the basis of calcium sulfate dihydrate and at least one member of the group consisting of calcium carbonate, β-tricalcium phosphate, calcium hydrogen phosphate, calcium hydrogen phosphate dihydrate, magnesium carbonate, trimagnesium phosphate, magnesium hydroxide and magnesium oxide
and at least one representative of the aminoglycoside antibiotics, lincosamide antibiotics, glycopeptide antibiotics, macrolide antibiotics, ketolide antibiotics, nitroimidazoles, fluorochinolone antibiotics, oxazolidinone antibiotics, the steroid antibiotics, the antiseptic agents and the fungicidal/fungistatic antibiotics,
with the granules being partly or entirely sheathed with a layer of glycerol tripalmitate and/or glycerol tristearate and/or glycerol trilaurate and/or 1-hexadecyl alcohol which connects the individual granules with each other and the granules being pressed to form a compact body which has no macropores of pore diameters larger than 50 µm.

2. Antibiotic/antibiotics containing bone substitute material according to claim 1 **characterized in that** it has the form of tablets of granules with a particle diameter of 100 to 2,500 µm or the form of granulate of granules with a particle diameter of 100 to 2,500 µm.

3. Bone substitute material according to claim 2, with the pellets being cylindrical or facetted.

4. Antibiotic/antibiotics containing bone substitute material according to one of claims 1 to 3, **characterized by** a composition with
50.0 - 90.0 percent by mass of calcium sulfate dihydrate,
1.0 - 40.0 percent by mass of calcium carbonate,
1.0 - 40.0 percent by mass of glycerol tripalmitate and/or glycerol tristearate and/or glycerol trilaurate and/or 1-hexadecyl alcohol, and
0.1 - 10.0 percent by mass of at least one representative of the aminoglycoside antibiotics, the lincosamide antibiotics, the glycopeptide antibiotics, the macrolide antibiotics, the ketolide antibiotics, the nitroimidazoles, the fluorochinolone antibiotics and the oxazolidinone antibiotics, the antiseptic agents, the steroid antibiotics and the fungistatic/fungicidal antibiotics.

5. Antibiotic/antibiotics containing bone substitute material according to one of claims 1 to 4, **characterized in that** the representative is gentamicin, tobramycin, netilmicin, sisomycin, amikacin, lincosamin, clindamycin, teicoplanin, vancomycin, ramoplanin, erythromycin, telithromycin, azithromycin, ciprofloxacin, moxifloxacin, linezolid, metronidazol, tinidazol, fluconazol, amphotericin B, nystatin, griseofulvin, fusidic acid, fosfomycin, rifampicin, chloramphenicol, chlorhexidine, polyhexanide.

## Revendications

1. Matériau de comblement osseux contenant un antibiotique et/ou des antibiotiques avec libération retardée du principe actif, **caractérisé en ce qu'**il est formé d'un mélange densifié de granules à base de sulfate de calcium dihydraté et d'au moins un élément du groupe constitué du carbonate de calcium, du phosphate de β-tricalcium, de l'hydrogénophosphate de calcium, de l'hydrogénophosphate de calcium dihydraté, du carbonate de magnésium, du phosphate de trimagnésium, de l'hydroxyde de magnésium et de l'oxyde de magnésium
et d'au moins un représentant des antibiotiques de type aminosides, des antibiotiques de type lincosamides, des antibiotiques de type glycopeptides, des antibiotiques de type macrolides, des antibiotiques de type kétolides, des nitro-imidazoles, des antibiotiques de type fluoroquinolones, des antibiotiques de type oxazolidinones, des antibiotiques stéroïdiens, des antiseptiques et des antibiotiques fongicides/fongistatiques,
étant entendu que les granules sont enveloppées partiellement ou totalement d'une couche de tripalmitate de glycérol et/ou de tristéarate de glycérol et/ou de trilaurate de glycérol et/ou d'alcool 1-hexadécylique qui lie les granules entre elles, et
les granules sont compressées en un corps compact qui ne possède pas de macropores ayant un diamètre de pore supérieur à 50 µm.

2. Matériau de comblement osseux contenant un antibiotique/des antibiotiques selon la revendication 1, **caractérisé en ce qu'**il se présente sous la forme de comprimés constitués de granules ayant un diamètre de particule de 100 à 2 500 µm ou sous la forme d'un granulat constitué de granules ayant un diamètre de particule de 100 à 2 500 µm.

3. Matériau de comblement osseux selon la revendication 2, où les comprimés sont cylindriques ou facettés.

4. Matériau de comblement osseux contenant un antibiotique/des antibiotiques selon l'une des revendications 1 à 3, **caractérisé par** une composition comportant
50,0 à 90,0 % en masse de sulfate de calcium dihydraté,
1,0 à 40,0 % en masse de carbonate de calcium,
1,0 à 40,0 % en masse de tripalmitate de glycérol et/ou de tristéarate de glycérol et/ou de trilaurate de glycérol et/ou d'alcool 1-hexadécylique, et
0,1 à 10,0 % en masse d'au moins un représentant des antibiotiques de type aminosides, des antibiotiques de type lincosamides, des antibiotiques de type glycopeptides, des antibiotiques de type macrolides, des antibiotiques de type kétolides, des nitro-imidazoles, des antibiotiques de type fluoroquinolones et des antibiotiques de type oxazolidinones, des antiseptiques, des antibiotiques stéroïdiens et des antibiotiques fongistatiques/fongicides.

5. Matériau de comblement osseux contenant un antibiotique/des antibiotiques selon l'une des revendications 1 à 4, **caractérisé en ce que** le représentant est la gentamicine, la tobramycine, la nétilmicine, la sisomycine, l'amikacine, la lincosamine, la clindamycine, la teicoplanine, la vancomycine, la ramoplanine, l'érythromycine, la télithromycine, l'azithromycine, la ciprofoxacine, la moxifloxacine, le linézolide, le métronidazole, le tinidazole, le fluconazole, l'amphotéricine B, la nystatine, la griséofulvine, l'acide fusidique, la fosfomycine, la rifampicine, le chloramphénicol, la chlorhexidine, le polyhexanide.
